# EUROPEAN PATENT SPECIFICATION

(11) **EP 4 066 754 B1**
(45) Date of publication and mention of the grant of the patent: **13.09.2023**
(21) Application number: 20828993.4
(22) Date of filing: 26.11.2020
(51) Int. Cl.: A61B 17/17, A61B 17/88, A61B 17/90

(54) **SURGICAL GUIDING DEVICE FOR BONE PERFORATION DURING OSTEOSYNTHESIS PROCESSES, ESPECIALLY FOR CORONOID FRACTURES OF THE ULNA**
CHIRURGISCHE FÜHRUNGSVORRICHTUNG ZUR KNOCHENPERFORATION BEI OSTEOSYNTHESEVERFAHREN, INSBESONDERE BEI CORONOIDFRAKTUREN DER ULNA
DISPOSITIF CHIRURGICAL DE GUIDE POUR PERFORATION OSSEUSE PENDANT DES PROCESSUS D'OSTÉOSYNTHÈSE PARTICULIÈREMENT POUR DES FRACTURES DE CORONOÏDES DE L'ULNA

(30) Priority: 26.11.2019 ES 201931040
(43) Date of publication of application: 05.10.2022
(73) Proprietor: Administración General de la Comunidad Autónoma de Euskadi, 01010 Vitoria-Gasteiz (Álava) (ES)
(72) Inventor: CEARRA GUEZURAGA, Iñigo, 01010 Vitoria- Gasteiz (Álava) (ES)
(74) Representative: Pons
(86) International application number: PCT/ES2020/070740
(87) International publication number: WO 2021/105541

(56) References cited:
- WO-A1-2018/198119
- GB-A- 2 506 373
- US-A1- 2011 224 734
- US-A1- 2013 079 788
- US-A1- 2017 209 192
- US-B1- 9 820 759

## Description

### OBJECT OF THE INVENTION

The present invention describes a surgical guiding device for bone perforation in osteosynthesis processes, especially in coronoid fractures of the ulna. More particularly, a spring-assisted, manually operated clamp-type device is described, which consists of an interchangeable guide head and a ring that acts as a referential fixed head, both with a toothed seat profile. This device enables maintaining the reduction of the fracture, of any size or morphology in the case of the coronoid of the ulna, in open surgery in a stable, automatic and intuitive way, and it serves as a guide for the osteosynthesis processes of the fracture. This device can further be used by just one surgeon with just one hand.

### BACKGROUND OF THE INVENTION

The coronoid process is a portion of the ulna that is fundamental for elbow stability, since it is a primary stabiliser thereof, especially preventing posterior and medial displacement of the ulna with respect to the humerus.

In certain elbow injury patterns, in particular those involving the dislocation thereof, the coronoid may be fractured. In the event of fractures involving over 50% of the coronoid height, coronoid osteosynthesis by means of different procedures is strongly agreed as the best solution. In fractures involving a smaller volume of the coronoid, fragment osteosynthesis as the best solution will depend on factors such as the morphology of the fragment, the association or lack thereof of other bone or ligament injuries, and in general the surgeon's final discretion. To carry out coronoid osteosynthesis, in most cases suture between bones or fixation with one or two screws is usually applied; this requires perforation from the dorsal side of the ulna, just on the other side of the bone.

This perforation starts from said dorsal side and it is necessary to progress by being oriented in space in order to exit into the fracture bed, with very little surface area and therefore with little margin of error. This makes the process difficult and sometimes the exit is not performed in the appropriate place.

In the state of the art, there are various devices with some connection to the described scenario; however, the existence of guides that facilitate the bone perforation process at the clinical level, such as those that exist in other anatomical regions and techniques, is unknown, such as the guide for performing an ACL (anterior cruciate ligament) plasty on the knee. In fact, some surgeons have precisely used paediatric knee ACL guides for synthesising coronoid fractures, trying to alleviate this deficit. However, the size of these guides is too large and the use thereof is uncomfortable, and less precise, at the elbow. Likewise, just one surgeon is unable to handle the guide and introduce a needle or drill bit at the same time, at least two people being essential to carry this out.

Thus, during osteosynthesis processes in coronoid fractures of the ulna, most of the time perforation is performed "by guessing", based on the discretion and experience of the surgeon, or occasionally using other types of guides designed for other anatomical regions, which fail to properly adapt.

With reference to the state of the art, some guides for coronoid osteosynthesis are described, although they are only designed for performing it by means of threads and stitches between bones (usually called the Lasso technique). For example, Chinese utility model CN204683756U discloses a device specifically designed for coronoid osteosynthesis, exclusively using threads and stitches between bones (Lasso technique). It is not designed for performing another essential type of osteosynthesis, with one or two screws. Said device discloses an auxiliary bar to the main arch designed for placing a cross bar, whose ends the suture threads pass through once they have passed through the coronoid fragment or through the anterior capsule, and before entering the tunnels between bones via the proximal ulna. This entails significant difficulties due to having to work in a very narrow space, where trying to position the auxiliary bar with the cross bar in said space is highly complicated.

There are also guides described only for application thereof in arthroscopic surgery, and only for apical coronoid fractures, such as model CN204158482 (U). It does not envisage the osteosynthesis of other types of fracture.

The devices that have already been described have many different parts assembled together, and consequently, greater difficulty for assembly and disassembly, in addition to hindering sterilisation and further complicating the use thereof, the surgeon requiring the help of an assistant for assembling and using the guide. Furthermore, the ends of the devices designed for holding the tip of the coronoid do not have an optimal design for keeping comminuted fractures reduced, nor a profile described as toothed or rough for providing greater stability and preventing movements.

US 2011/224734 discloses a compression plate kit that allows for manual compression control of a bone discontinuity for improved repair of fractures, fusions, and other bone discontinuities. The kit includes a compression clamp comprising a first lever secured to a second lever via a pivot. Furthermore, the compression clamp includes a biasing mechanism configured to bias the ends of the first lever away from the ends of the second lever. This document discloses the closest prior art to the subject-matter of appended claim 1.

### DESCRIPTION OF THE INVENTION

The present invention seeks to solve some of the problems mentioned in the state of the art. More particularly, a device is described that enables bone perforation of the ulna and coronoid to be performed in a precise way, further enabling the use of different drill bit or needle thicknesses, thus performing tunnels for sliding stitches between bones, or one or more tunnels for placing one or more osteosynthesis screws. This device can be used by just one surgeon, and enables maintaining the reduction and perforating, from the proximal ulna, fragments of different sizes and morphology, therefore being versatile for any type of coronoid fracture and enabling precise osteosynthesis with stitches, needles and/or screws to be performed.

More particularly, a surgical guiding device for bone perforation during osteosynthesis processes is described, especially for coronoid fractures of the ulna, comprising:
- a first tightening arm which at a distal end comprises a first ring intended for resting on a fractured area,
- a second tightening arm which at a distal end comprises a second ring intended for resting on the dorsal area of the proximal ulna, said second ring joined to a head, wherein each arm comprises an intermediate portion which in turn comprises respective widened sections integrated with each other by means of a shaft or rod concentric to said widened sections, enabling a controlled clamp-type movement between said arms, and
- a spring joined at a proximal end of each arm by means of respective extensions and configured for exerting traction on the proximal ends of both arms and consequently exerting compression on the distal ends, enabling the rings to exert pressure on the fractured area, preventing movements.

The described device automatically compresses the fracture and maintains the reduction of the coronoid by means of the spring without the need to loosen nuts or manually compress each time the interchangeable head that can be coupled to the second ring needs to be changed. This make it possible for just one surgeon to handle it with one hand.

The spring is configured for exerting traction on the proximal end of both arms and exerting compression on the distal end thereof, so that the first and second rings therein exert pressure on the coronoid and the dorsal face of the ulna once they are placed in position for perforation, stabilising the reduction of the fracture and preventing movements.

Preferably, the head that acts as a guide for perforation is interchangeable and removable, with the possibility of different reduction diameters, enabling bone tunnels of different diameters to be performed, and making it possible to synthesise fractures of any size by means of open surgery, using needles, stitches between bones and/or screws. Likewise, both rings on both arms can have a toothed profile for increasing the friction on the coronoid and the back of the ulna, respectively, and for preventing sliding and/or loss of reduction.

The device can further have, in the intermediate portion, an ergonomic wheel threaded on the rotation shaft or rod and integrated with the widened section of the arms, intended for being comfortably rotated by the surgeon to exert pressure on the coronoid in the position required for bone perforation. More specifically, said ergonomic wheel can rotate on the threaded rod and be tightened so that it acts as an adjustable brake, and it can fix the relative position of the two arms at any point in the range of motion.

Likewise, the device may comprise two internal stops on the first arm and on the second arm for delimiting the minimum distance between the two arms, reducing unnecessary degrees of travel and favouring manoeuvrability.

Preferably, the first ring has a wider circular configuration, i.e., of greater diameter, than height or thickness for favouring visibility and manoeuvrability on the coronoid.

Note that the device may have a very reduced number of parts, joined together forming a simple mechanism, without the need for assembling and disassembling, which enables it to be operated by just one surgeon and favouring sterilisation, maintenance, manoeuvrability and visibility. Likewise, the device is versatile and enables fractures of any size to be synthesised by means of open surgery.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1 shows a rear perspective view of a preferred embodiment of the surgical device for bone perforation during osteosynthesis processes in coronoid fractures of the ulna.
Figure 2 shows a front perspective view of a preferred embodiment wherein the surgical device is shown being used from a surgeon's perspective during the osteosynthesis process and placed on the coronoid for bone perforation.
Figure 3 shows an exploded view of a preferred embodiment, wherein the different parts that make up the surgical device are shown.

### PREFERRED EMBODIMENT OF THE INVENTION

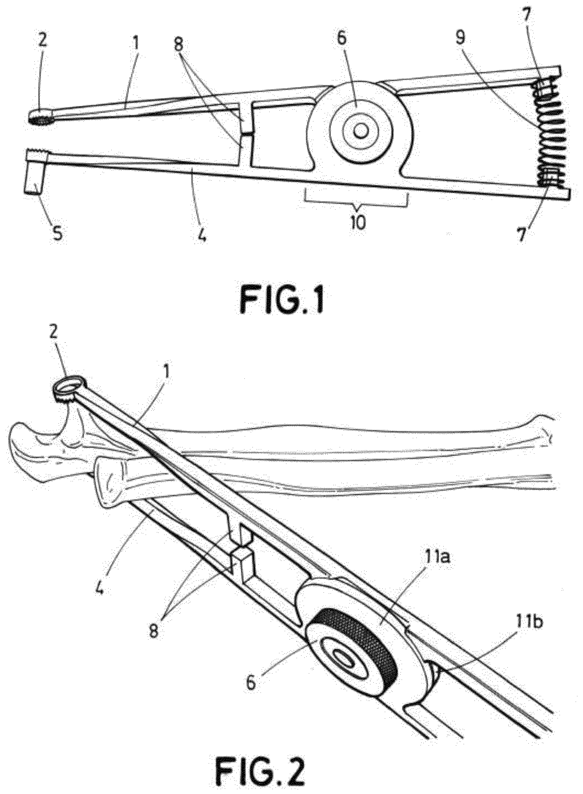

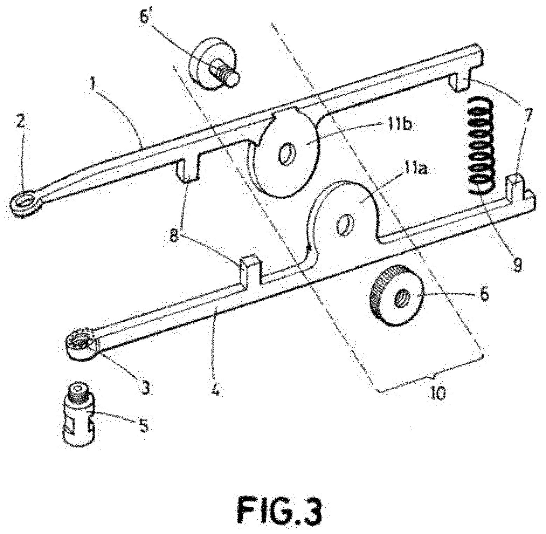

Figures 1 and 2 show different perspective views of a preferred embodiment of the surgical device for bone perforation during osteosynthesis processes in coronoid fractures of the ulna. Figure 3 shows an exploded view of a preferred embodiment. Said surgical device comprises a first tightening arm (1), at the distal end of which a first ring (2) is housed, which is intended for resting on the upper area of the coronoid.

Likewise, Figures 1, 2 and 3 show that the device comprises a second tightening arm (4) which incorporates a second ring (3), in this case threaded, in the distal portion thereof onto which an interchangeable head (5)with different reduction diameters can be screwed, which makes it possible to slide and guide needles or drill bits for performing bone tunnels, and it can further guide the insertion of one or more screws for osteosynthesis.

In the preferred embodiment described by Figures 1, 2 and 3, the head (5) is interchangeable and removable for performing tunnels of different diameters and synthesising fractures of any size or morphology with needles, screws or suture between bones by means of open surgery. Additionally, in said embodiment the first ring (2) and second ring (3) each have a mutually opposite toothed profile for increasing the friction on the coronoid and the back of the proximal ulna and for preventing sliding or loss of reduction of the fracture during surgery.

Likewise, the first ring (2) has a circular morphology of greater width (diameter) than height, in the direction of the greater shaft of the coronoid, for favouring visibility and manoeuvrability, due to the narrowness of the work space available during surgery.

Figure 2 shows a front perspective view from the perspective or point of view of a surgeon of the described preferred embodiment, wherein the surgical device is shown being used and placed on the coronoid for bone perforation and Figure 3 shows an exploded view of a preferred embodiment. Specifically, Figures 2 and 3 show that the device further comprises an intermediate portion (10) which comprises respective widened sections (11a, 11b) in both arms (1, 4) with a circular configuration or, for example, a washer shape, which are integrated around a shaft or rod (6') in the centre of said circles or washers, thus enabling a controlled clamp-type movement between the arms (1, 4). Said rod (6') is threaded and comprises an ergonomic wheel (6), which enables the degree of sliding and the stable fixation between both arms (1, 4) to be adjusted and acts as an adjustable brake, and it can fix the relative position of the two arms (1, 4) at any point in the range of motion.

Additionally, it is clearly shown that the device comprises a spring (9), which is housed between the proximal ends of both arms via the extensions (7) provided for this purpose, which exerts a separation force on said proximal ends of the arms (1, 4), and an approaching force on the distal ends of the arms (1, 4) and the respective rings (2 and 3) thereof, such that they exert pressure on the coronoid and on the back of the ulna once they are placed in position for perforation, thus preventing movements or loss of reduction of the fracture, and enabling the perforation and osteosynthesis process to be performed intuitively, easily, and by just one surgeon.

Additionally, in the preferred embodiment, each arm of the device (1, 4) has respective extensions (7) which act by way of a stop (8) for delimiting a minimum opening between the two rings (2, 3) of the distal ends of the arms (1, 4), preventing unnecessary degrees of travel and, ultimately, friction between the same and the deterioration thereof.

## Claims

1. A surgical guiding device for bone perforation during osteosynthesis processes, especially for coronoid fractures of the ulna, **which** comprises:
- a first tightening arm (1) which at a distal end comprises a first ring (2) intended for resting on a fractured area,
- a second tightening arm (4) which at a distal end comprises a second ring (3) intended for resting on the dorsal area of the proximal ulna, said second ring (3) joined to a head (5), wherein each arm (1, 4) comprises an intermediate portion (10) which in turn comprises respective widened sections (11a, 11b) connected with each other by means of a rod (6') concentric to said widened sections (11a, 11b), enabling a controlled clamp-type movement between said arms (1, 4),
- a spring (9) joined to a proximal end of each arm (1, 4) by means of respective extensions (7)
**characterised in that** the spring (9) is configured for pulling apart the proximal ends of both arms (1, 4) and consequently exerting compression on the distal ends, enabling the rings (2, 3) to exert pressure on the fractured area, preventing movements, and **in that** it further comprises:
- an ergonomic wheel (6) adapted for threading with the rod (6'), thus acting as a brake and enabling the arms (2, 3) to be tightened and fixed in a desired opening position.

2. The surgical device of claim 1, wherein the head (5) is interchangeable and removable by means of a threaded joint with the second ring (3).

3. The surgical device of claim 1, wherein the rings (2, 3) each have a mutually opposite toothed profile.

4. The surgical device of claim 1, wherein the first ring (2) has a circular configuration with a diameter greater than the height or thickness of said first ring (2) for favouring visibility and manoeuvrability.

5. The surgical device of claim 1, wherein the first arm (1) and the second arm (4) comprise two stops (8) for delimiting a minimum opening position of the clamp.

## Patentansprüche

1. Chirurgische Führungsvorrichtung zur Knochenperforation bei Osteosyntheseverfahren, insbesondere bei Coronoidfrakturen der Ulna, **welche** umfasst:
- einen ersten Spannarm (1), der an einem distalen Ende einen ersten Ring (2) umfasst, der dazu bestimmt ist, auf einem gebrochenen Bereich aufzuliegen,
- einen zweiten Spannarm (4), der an einem distalen Ende einen zweiten Ring (3) umfasst, der dazu bestimmt ist, auf dem dorsalen Bereich der proximalen Ulna zu ruhen, wobei der zweite Ring (3) mit einem Kopf (5) verbunden ist, wobei jeder Arm (1, 4) einen Zwischenabschnitt (10) umfasst, der seinerseits entsprechende verbreiterte Abschnitte (11a, 11b) umfasst, die miteinander mittels einer Stange (6') verbunden sind, die konzentrisch zu den verbreiterten Abschnitten (11a, 11b) ist, was eine kontrollierte klemmartige Bewegung zwischen den Armen (1, 4) ermöglicht,
- eine Feder (9), die mit einem proximalen Ende jedes Arms (1, 4) mittels entsprechender Verlängerungen (7) verbunden ist, **dadurch gekennzeichnet, dass** die Feder (9) konfiguriert ist, um die proximalen Enden beider Arme (1, 4) auseinander zu ziehen und folglich Druck auf die distalen Enden auszuüben, was es den Ringen (2, 3) ermöglicht, Druck auf den gebrochenen Bereich auszuüben, wodurch Bewegungen verhindert werden, und **dadurch, dass** sie ferner umfasst:
- ein ergonomisches Rad (6), das sich in die Stange (6') einfädeln lässt und so als Bremse wirkt und das Festziehen und Fixieren der Arme (2, 3) in einer gewünschten Öffnungsposition ermöglicht.

2. Chirurgische Vorrichtung nach Anspruch 1, wobei der Kopf (5) durch eine Schraubverbindung mit dem zweiten Ring (3) austauschbar und abnehmbar ist.

3. Chirurgische Vorrichtung nach Anspruch 1, wobei die Ringe (2, 3) jeweils ein einander gegenüberliegendes Zahnprofil aufweisen.

4. Chirurgische Vorrichtung nach Anspruch 1, wobei der erste Ring (2) eine kreisförmige Konfiguration mit einem Durchmesser aufweist, der größer ist als die Höhe oder Dicke des ersten Rings (2), um die Sichtbarkeit und Manövrierbarkeit zu verbessern.

5. Chirurgische Vorrichtung nach Anspruch 1, wobei der erste Arm (1) und der zweite Arm (4) zwei Anschläge (8) zur Begrenzung einer minimalen Öffnungsposition der Klemme umfassen.

## Revendications

1. Dispositif de guidage chirurgical pour le perçage d'os au cours de processus d'ostéosynthèse, en particulier pour des fractures de coronoïde de l'ulna, **qui** comprend :
- un premier bras de serrage (1) qui comprend à une extrémité distale une première bague (2) destinée à prendre appui sur une zone fracturée,
- un second bras de serrage (4) qui comprend à une extrémité distale une seconde bague (3) destinée à prendre appui sur la zone dorsale de l'ulna proximal, ladite seconde bague (3) étant liée à une tête (5), dans lequel chaque bras (1, 4) comprend une partie intermédiaire (10) qui à son tour comprend des sections élargies respectives (11a, 11b) reliées l'une à l'autre au moyen d'une tige (6') concentrique avec lesdites sections élargies (11a, 11b), permettant un mouvement contrôlé de type pince entre lesdits bras (1,4),
- un ressort (9) lié à une extrémité proximale de chaque bras (1, 4) au moyen de prolongements respectifs (7), **caractérisé en ce que** le ressort (9) est configuré pour écarter les extrémités proximales des deux bras (1, 4) et en conséquence exercer une compression sur les extrémités distales, permettre aux bagues (2, 3) d'exercer une pression sur la zone fracturée, empêcher des mouvements, et **en ce qu'**il comprend en outre :
- une molette ergonomique (6) adaptée à l'enfilage avec la tige (6'), agissant ainsi comme un frein et permettant de serrer et de fixer les bras (2, 3) dans une position d'ouverture souhaitée.

2. Dispositif chirurgical selon la revendication 1, dans lequel la tête (5) est interchangeable et amovible au moyen d'une liaison filetée avec la seconde bague (3).

3. Dispositif chirurgical selon la revendication 1, dans lequel les bagues (2, 3) ont chacune un profil denté opposé l'un à l'autre.

4. Dispositif chirurgical selon la revendication 1, dans lequel la première bague (2) a une configuration circulaire avec un diamètre supérieur à la hauteur ou à l'épaisseur de ladite première bague (2) pour favoriser la visibilité et la maniabilité.

5. Dispositif chirurgical selon la revendication 1, dans lequel le premier bras (1) et le second bras (4) comprennent deux butées (8) pour délimiter une position d'ouverture minimale de la pince.
